# EUROPÄISCHE PATENTSCHRIFT

(11) **EP 3 294 236 B1**
(45) Veröffentlichungstag und Bekanntmachung des Hinweises auf die Patenterteilung: **03.07.2019**
(21) Anmeldenummer: 16723335.2
(22) Anmeldetag: 12.05.2016
(51) Int. Cl.: A61F 5/01

(54) **VORRICHTUNG ZUM BEGRENZEN EINER BEWEGUNG EINES ZWISCHEN EINEM ERSTEN KÖRPERTEIL UND EINEM ZWEITEN KÖRPERTEIL ANGEORDNETEN GELENKS**
DEVICE FOR LIMITING A MOVEMENT OF A JOINT ARRANGED BETWEEN A FIRST BODY PART AND A SECOND BODY PART
DISPOSITIF PERMETTANT DE LIMITER UN MOUVEMENT D'UNE ARTICULATION AGENCÉE ENTRE UNE PREMIÈRE PARTIE CORPORELLE ET UNE DEUXIÈME PARTIE CORPORELLE

(30) Priorität: 12.05.2015 DE 102015107405
(43) Veröffentlichungstag der Anmeldung: 21.03.2018
(73) Patentinhaber: Betterguards Technology GmbH, 10625 Berlin (DE)
(72) Erfinder: BICHLER, Vinzenz, 10777 Berlin (DE)
(74) Vertreter: Okoampah, Rene
(86) Internationale Anmeldenummer: PCT/EP2016/060635
(87) Internationale Veröffentlichungsnummer: WO 2016/180913

(56) Entgegenhaltungen:
- EP-A1- 1 685 814
- DE-A1-102012 011 433
- US-A- 5 712 011
- US-A1- 2014 015 176

## Beschreibung

### Technisches Gebiet

Die Erfindung bezieht sich auf eine Vorrichtung zum Begrenzen einer Bewegung eines zwischen einem ersten Körperteil und einem zweiten Körperteil angeordneten Gelenks, wobei zur Bewegungsbegrenzung ein dilatantes Fluid verwendet wird.

### Stand der Technik

Schädigungen im Bereich der am Bewegungszyklus beteiligten Gelenke zählen zu einem der wichtigsten Unfallschwerpunkte. Dabei sind besonders die so genannten "Stolper-, Rutsch- und Sturzunfälle" (SRS-Unfälle) hervorzuheben, welche als Verletzungsursache bei Wege-, Arbeits- und Sportunfällen am häufigsten vorkommen. Diese Unfälle können einzeln oder in Kombination zu erheblichen Verletzungen führen. Überwiegend betroffen sind vor allem größere Gelenke, wie das obere und untere Sprunggelenk (articulatio talocuralis und articulatio talotarsalis), das Handgelenk (articulatio manus) sowie das Kniegelenk (articulatio genus). Dabei ist eine Verletzungstendenz in diesem Bereich auch sportmedizinisch zu verzeichnen.

Durch die SRS-Unfälle kommt es häufig zu einer gewaltsamen Überdehnung des Halteapparats des Gelenks, oftmals in Supinationsrichtung. Die Verletzungsursachen sind meist hohe Supinations- (Inversions-) Geschwindigkeit, sowie ein Überschreiten eines für die Verletzung verantwortlichen kritischen Winkels, bspw. beim Umknicken am Sprunggelenk. So kann bspw. eine kritische Winkelgeschwindigkeit beim Umknicken am Sprunggelenk von über ca. 300/s zu einer Verletzung führen. Im Folgenden werden Kennwerte unterhalb dieser kritischen Winkelgeschwindigkeit bei Ausführungsvarianten am Sprunggelenk als physiologische Bewegungen bezeichnet. Oberhalb dieses kritischen Kennwertes werden Bewegungen als unphysiologische Bewegungen bezeichnet. Die auftretenden Geschwindigkeiten sind auf Kräfte beziehungsweise Beschleunigungen zurückzuführen, welche auf die Körperteile und Gelenke wirken.

Ein sehr wichtiger Faktor für den positiven Heilungsverlauf eines Supinationstraumas ist die Stabilisierung der Gelenke des Patienten, zur Verletzungsprävention oder während der prä- oder postoperativen Versorgung. Dabei wird die Stabilisierung der Gelenke in der Regel über Tapeverbände, Funktionssicherungsorthesen, Stabilisierungsorthesen, Bandagen und/oder stabile Schuhe mit variabler Schafthöhe und optionalen Verstärkungselementen erreicht.

Dabei kommen insbesondere dynamische Orthesen zur Regulierung der Kraft zwischen relativ zueinander bewegbaren Körperteilen zum Einsatz. Bestehende Vorrichtungen ermöglichen bisher entweder eine starke Schutzwirkung mit damit einhergehender Immobilisierung, oder aber eine leichte Schutzwirkung, die bei einem Verletzungsfall nur unzureichend schützt. Für verschiedene Lastszenarien ist somit ein Wechsel der Vorrichtung beziehungsweise eine manuelle Voreinstellung nötig, um die Eigenschaften der Vorrichtung dem Heilungsverlauf anzupassen.

Unter anderem sind auch Orthesen bekannt, deren Beweglichkeit von dem Scherverhalten eines dilatanten Fluids abhängt. Für eine adaptive Bewegungsbegrenzung kommen dilatante, scherverdickende Materialien, wie beispielsweise Copolymerdispersionen oder -fluide, welche bei dem Auftreten von hohen Scherkräften und einer damit verbundenen hohen Schwergeschwindigkeit eine höhere Viskosität aufweisen, zum Einsatz. Je größer die aufgebrachte Scherung ist, desto viskoser beziehungsweise zähflüssiger verhält sich das Fluid. Entsprechend werden dilatanten Fluiden auch scherverfestigende beziehungsweise scherverdickende Eigenschaften zugeschrieben.

Orthesen, welche sich die Eigenschaften solcher dilatanten Fluide zu Eigen machen, können beispielsweise eine Aufnahme und einen Auszugskörper aufweisen. Dabei ist die Aufnahme mit dem dilatanten Fluid gefüllt. Der Auszugskörper ragt in die Aufnahme hinein, sodass zumindest der mit der Aufnahme überlappende Bereich des Auszugskörpers von dem dilatanten Fluid umgeben ist. Der Auszugskörper kann dabei an einem ersten Körperteil angebracht sein, die Aufnahme hingegen an einem sich zu dem ersten Körperteil relativ bewegbaren zweiten Körperteil.

Bei einer geringen Kräfteeinwirkung kann der Auszugskörper in dem dilatanten Fluid nahezu uneingeschränkt bewegt werden. Bei hoher Krafteinwirkung und einem daraus resultierenden starken beziehungsweise abrupten Zug am Auszugskörper kommt es zu einem Überschreiten der kritischen Schergeschwindigkeit in dem dilatanten Fluid. Dies hat je nach verwendetem Fluid einen Anstieg der Zugspannung um den Faktor 100 bis zum Faktor 1000 zur Folge. Der Anstieg der Zugspannung hat eine vernetzende Wirkung auf das dilatante Fluid, wodurch der eine hohe Krafteinwirkung erfahrende Auszugskörper durch den starken Anstieg der Viskosität des dilatanten Fluids im selbigen gehalten wird und so keine oder nur eine verminderte Bewegung erfährt. Hierdurch ist es prinzipiell möglich, physiologische Bewegungen ohne Verletzungsgefährdung des zu stabilisierenden Gelenks beziehungsweise deren Stützapparat, wie Sehnen, Bänder, Knorpel und Muskeln, zu ermöglichen, da hierbei nur geringe Beschleunigungen und Kräfte wirken. Bei einer unphysiologischen Bewegung, bei welcher eine Verletzung des Gelenks und/oder dessen Stützapparats auftreten kann, kann die Bewegung des Gelenks durch die mittels des dilatanten Fluids erzeugten Haltewirkung des Auszugskörpers in der Aufnahme begrenzt werden und somit das Gelenk beziehungsweise der Stützapparat vor einer Verletzung geschützt werden.

Die US 5,712,011 zeigt beispielsweise eine zerrresistente Einheit, wobei ein erster Teil einen nichtdehnbaren Abschnitt mit einer Oberfläche aufweist, welche mit der Oberfläche eines zweiten, nichtdehnbaren Teils überlappt. Die beiden Teile sind dabei derart angeordnet, dass sie eine translatorische, im Wesentlichen parallele Bewegung relativ zueinander zulassen. Zwischen den Teilen ist eine scherverfestigende Zusammensetzung angeordnet, welche einen variablen Widerstand gegen die translatorische Bewegung des zweiten Teils relativ zu dem ersten Teil aufbringt. Dabei resultiert der Widerstand aus den auf die scherverfestigende Zusammensetzung wirkenden Scherkräfte, welche sich aus der Relativbewegung der nichtdehnbaren Teile zueinander ergeben. Die beiden überlappenden Teile sowie die scherverfestigende Zusammensetzung sind dabei von einer Hülle umgeben.

In der DE102012011433 A1 ist eine Vorrichtung zur adaptiven Bewegungsbegrenzung insbesondere für Orthesen und Bandagen mit einem mit dilatantem Fluid befüllten Behälter und einem in dem Behälter bewegbaren Körper gezeigt. Durch das dilatante Fluid ist eine sich den wirkenden Geschwindigkeiten, Beschleunigungen und Kraftintensitäten anpassende, adaptive Bewegungsbegrenzung des Körpers bezüglich des Behälters in einer Auszugsrichtung des Körpers ermöglicht. Bei einer Zugbelastung auf die Vorrichtung, beispielsweise bei einer Umknickbewegung, wird der bewegbare Körper aus der Aufnahme gezogen, wobei das dilatante Fluid ab einer kritischen Schergeschwindigkeit das Ausziehen des Körpers behindert beziehungsweise verhindert. Um einen konstanten Scherspalt zwischen der Innenwand der Aufnahme und der Oberfläche des bewegbaren Körpers bereitzustellen, sind an dem bewegbaren Körper Abstandhalter angeordnet.

Nachteilig im derzeitigen Stand der Technik ist unter anderem, dass sich durch das Ausziehen des bewegbaren Körpers aus der Aufnahme die effektiv scherkraftübertragende Fläche des bewegbaren Körpers mit zunehmendem Hub beziehungsweise Auszugsweg verringert. So ist mit zunehmendem Gelenkwinkel eine abnehmende Rückhaltekraft bereitgestellt. Kommt es bei einem Ausziehen eines Auszugskörpers zu einem scherverfestigenden Effekt, ist das dilatante Fluid zumindest an den Stellen der Scherverfestigung nicht mehr in der Lage, das durch den ausgezogenen Auszugskörper frei gewordene Volumen aufzufüllen, da durch die Scherverfestigung ein Nachfließen, wie es unterhalb der kritischen Schergeschwindigkeit bereitgestellt ist, verhindert wird.

### Darstellung der Erfindung

Ausgehend von dem bekannten Stand der Technik ist es eine Aufgabe der vorliegenden Erfindung, eine verbesserte Vorrichtung zum Begrenzen einer Bewegung eines zwischen einem ersten Körperteil und einem zweiten Körperteil angeordneten Gelenks bereitzustellen.

Diese Aufgabe wird mit einer Vorrichtung zum Begrenzen einer Bewegung eines zwischen einem ersten Körperteil und einem zweiten Körperteil angeordneten Gelenks mit den Merkmalen des Anspruch 1 gelöst. Vorteilhafte Weiterbildungen ergeben sich aus den Unteransprüchen.

Entsprechend wird eine Vorrichtung zum Begrenzen einer Bewegung eines zwischen einem ersten Körperteil und einem zweiten Körperteil angeordneten Gelenks angegeben, welche eine mittels einer Aufnahmebefestigung an dem ersten Körperteil gehaltenen Aufnahme, einen mittels einer Scherkörperbefestigung an dem zweiten Körperteil gehaltenen, relativ zu der Aufnahme bewegbaren Scherkörper, wobei der Scherkörper über eine Einführöffnung zumindest teilweise in die Aufnahme eingeführt ist, und ein in der Aufnahme bereitgestelltes dilatantes Fluid zur Beeinflussung einer Relativbewegung zwischen dem Scherkörper und der Aufnahme, umfasst. Erfindungsgemäß ist die Einführöffnung der Aufnahme von der Scherkörperbefestigung weg gerichtet.

Dadurch, dass die Einführöffnung der Aufnahme von der Scherkörperbefestigung weg gerichtet ist, ist es möglich, dass eine Zugbelastung auf das Gelenk beziehungsweise auf den der Vorrichtung zugewandten Teil des Stützapparats des Gelenks in einer mittels des dilatanten Fluids übertragenen, zwischen dem Scherkörper und der Aufnahme wirkenden Druckbelastung resultiert. Mit anderen Worten bewirkt eine Vergrößerung des Winkels zwischen dem ersten Körperteil und dem zweiten Körperteil, beispielsweise bei einem Umknicken am Sprunggelenk, ein Auseinanderbewegen der Aufnahmebefestigung und der Scherkörperbefestigung, wodurch der Scherkörper in die Aufnahme bewegt beziehungsweise gedrückt wird.

Durch ein Eindrücken des Scherkörpers in die Aufnahme kann gegenüber den aus dem Stand der Technik bekannten Ausführungen mit Auszugskörper bei gleichen Abmessungen, gleichen Materialien und gleichen Belastungen eine verbesserte dilatante Wirkung der Vorrichtung bereitgestellt werden. So ist der dilatante Effekt bei einem Eindrücken des Scherkraftkörpers in die Aufnahme höher, als bei einem Ausziehen des Auszugkörpers aus der Aufnahme. Bei einem Eindrücken des Körpers in das dilatante Fluid kommt es zu keinem oder verglichen zum Ausziehen in geringerem Maße zu einem Ablösen der Wandhaftung des dilatanten Fluids von der Oberfläche des Scherkörpers beziehungsweise der Innenwand der Aufnahme.

Ferner nimmt die effektiv scherkraftübertragene Fläche des Scherkörpers mit zunehmender Einführtiefe in der Aufnahme zu. Somit kann bei einem Eindrücken des Scherkörpers eine mit zunehmender Einführtiefe zunehmende dilatante Wirkung der Vorrichtung bereitgestellt werden, was die Rückhaltekraft der Vorrichtung gegen ein Auseinanderbewegen der beiden Befestigungen, wie beispielsweise bei einem Umknicken am Sprunggelenk, verstärkt. So kann die Vorrichtung mit einer mit zunehmender Eindringtiefe ansteigenden Bewegungsdämpfung bereitgestellt werden.

Aufgrund des sich stärker ausprägenden dilatanten Effekts beziehungsweise der stärkeren Rückhaltung und Dämpfung der Bewegung des Scherkörpers beim Eindrücken kann die Vorrichtung mit einem besonders niederviskosen dilatanten Fluid versehen sein. So kann eine besonders leichtgängige Vorrichtung bereitgestellt werden, wobei das dilatante Fluid bevorzugt nur im Lastfall, folglich bei unphysiologischen Bewegungen, scherverfestigt.

Bevorzugt kann als dilatantes Fluid ein dilatantes beziehungsweise scherverdickendes Fluid Anwendung finden. Alternativ können beispielsweise auch Gele, Pasten, aber auch Wirkmedien mit Feststoffcharakter oder Feststoffe mit dilatanten beziehungsweise scherverfestigenden Eigenschaften, wie Schäume, als dilatantes Fluid verwendet werden.

In einer bevorzugten Weiterbildung ist zwischen dem Scherkörper und der Scherkörperbefestigung ein U-förmiger Verbindungsbereich zum Verbinden des Scherkörpers mit der Scherkörperbefestigung angeordnet. Dadurch kann eine einfache Umlenkung der auf die über die Aufnahmebefestigung und die Scherkörperbefestigung auf die Vorrichtung aufgebrachten Kräfte bereitgestellt werden. So kann eine auf das Gelenk beziehungsweise auf den der Vorrichtung zugewandten Teil des Stützapparats des Gelenks wirkende Zugbelastung, welche über die Aufnahmebefestigung und die Scherkörperbefestigung eingeleitet und hierdurch auf die Vorrichtung übertragen wird, als eine Druckbelastung in das dilatante Fluid eingebracht werden. Ferner kann die Vorrichtung durch den U-förmigen Verbindungsbereich besonders kompakt ausgeführt werden, was insbesondere für die Verwendung im Sportbereich von Vorteil ist.

Bevorzugt ist der Scherkörper mit dem U-förmigen Verbindungsbereich einteilig bereitgestellt. Alternativ können der Scherkörper und der Verbindungsbereich auch aus mehreren Teilen bestehen und miteinander verbunden sein.

In einer weiter bevorzugten Ausführungsform sind die Aufnahmebefestigung und die Scherkörperbefestigung derart angeordnet, dass bei an dem ersten Körperteil angebrachter Aufnahmebefestigung und bei an dem zweiten Körperteil angebrachter Scherkörperbefestigung die Einführöffnung proximal oder kranial an der Aufnahme angeordnet ist.

Dadurch ist es möglich, dass die Einführöffnung bei am Körper angebrachter Vorrichtung an der Seite der Aufnahme angeordnet ist, welche in Ruhelage sowie bei Bewegung des Körpers im Wesentlichen der Erdanziehungskraft entgegen gerichtet ist. Somit wird eine an der Öffnung zwischen Scherkörper und Aufnahme angeordnete Dichtung zum Abdichten des Innenraums der Aufnahme im Wesentlichen nicht durch in Richtung der Dichtung fließendes beziehungsweise drückendes, in der Aufnahme befindliches dilatantes Fluid beeinflusst. Insbesondere, wenn die Dichtung als elastische Dichtung bereitgestellt ist und/oder wenn ein dilatantes beziehungsweise scherverdickendes Fluid als dilatantes Fluid verwendet wird, kann durch diese Anordnung der Öffnung ein Ansammeln des dilatanten Fluids im Bereich der Dichtung und ein damit einhergehendes Ausfließen des dilatanten Fluids aus dem Bereich der Aufnahme, welcher zur Scherkraftübertragung vorgesehen ist, verhindert werden.

In einer weiter bevorzugten Ausgestaltung verbindet der U-förmige Verbindungsbereich einen Interaktionsabschnitt des Scherkörpers, der mit dem dilatanten Fluid in Wirkverbindung steht, und einen Führungsabschnitt des Scherkörpers, welcher sich zur Scherkörperbefestigung erstreckt, wobei der Interaktionsabschnitt und der Führungsabschnitt sich zumindest teilweise parallel und voneinander beabstandet in Eindrückrichtung erstrecken und sich zumindest teilweise überlappen.

Entsprechend ist der Scherkörper in Abschnitte unterteilt, denen unterschiedliche Funktionen zukommen. Der Interaktionsabschnitt kann flach um breit ausgestaltet werden, um eine möglichst große Scherfläche bereitzustellen. Der Führungsabschnitt hingegen kann in beliebiger Form ausgebildet sein. Aufgabe des Führungsabschnitts ist es Kräfte zwischen dem Scherkörper und der Scherkörperbefestigung zu übertragen. Der U-förmige Verbindungsbereich ermöglicht die Umkehrung der Lastrichtung, wodurch an der Scherkörperbefestigung wirkende Zugkräfte in Form von Druckkräften von dem Interaktionsabschnitt in die Aufnahme eingebracht werden und an der Scherkörperbefestigung wirkende Druckkräfte in Form von Zugkräften von dem Interaktionsabschnitt in die Aufnahme eingebracht werden.

In einer weiter bevorzugten alternativen Ausführung ist ein Anschlag zum Begrenzen der Einführbewegung des Scherkörpers in die Aufnahme vorgesehen. Dadurch kann das zu schützende Gelenk in seiner Bewegung begrenzt werden. So kann verhindert werden, dass das Gelenk einen kritischen Winkel oder Weg überschreitet, ab dem Beeinträchtigungen, wie Verletzungen, auftreten können. Der Anschlag verhindert dabei ein Weiterbewegen des Scherkörpers in der Aufnahme. Bevorzugt ist der Anschlag am Boden der Aufnahme angeordnet und besonders bevorzugt ist der Boden der Aufnahme selbst als Anschlag vorgesehen.

In einer alternativen Ausführungsform kann der Anschlag auch am U-förmigen Verbindungsbereich angeordnet und/oder durch diesen ausgebildet sein. Dadurch ist es möglich den maximalen Bewegungsspielraum der Vorrichtung durch Verwenden von Scherkörpern mit unterschiedlichen Anschlägen einzustellen. Bei fortschreitender Heilung des Anwenders kann schrittweise von Scherkörpern, deren Anschläge kleine Bewegungen zulassen, zu Scherkörpern, deren Anschläge größerer Bewegung zulassen, gewechselt werden. Zur Einstellbarkeit nötig ist dabei lediglich der Austausch der Scherkörper mit entsprechendem Anschlag.

In einer Weiterbildung sind mindestens zwei Anschläge vorgesehen, wobei mindestens ein Anschlag zum Begrenzen der Auszugsbewegung des Scherkörpers aus der Aufnahme und mindestens ein Anschlag zum Begrenzen der Eindrückbewegung des Scherkörpers in die Aufnahme vorgesehen sind. Hierdurch kann die zu limitierende Gelenkbewegung in beide Bewegungsrichtungen begrenzt werden.

In einer alternativen Ausführungsform ist die Lage des Anschlags an der Aufnahme variabel. Mit anderen Worten kann die Position des Anschlags in der Aufnahme in beziehungsweise entgegen der Eindrückrichtung verändert werden. Hierdurch kann der Bewegungsumfang des Scherkörpers in der Aufnahme und somit die Gelenkbewegung variiert werden, beispielsweise um bei einer Anbringung der Vorrichtung an einer Orthese, einem Hilfsmittel, einem Sportprodukt, einem Schuh oder dergleichen den Bewegungsumfang entsprechend eines Genesungszustands anzupassen.

In einer weiter bevorzugten Ausführungsvariante ist der Scherkörper über eine externe Führung geführt, wobei die externe Führung außerhalb eines mit dem dilatanten Fluid gefüllten Bereichs der Aufnahme angeordnet ist. Dadurch kann eine Vorrichtung mit einer deutlich vergrößerten Leichtgängigkeit bei Geschwindigkeiten unterhalb der kritischen Schergeschwindigkeit bereitgestellt werden, da keine Abstandshalter notwendig sind, welche aufgrund ihres minimalen Wandabstands eine frühzeitige Scherverdickung hervorrufen würden. Die Führung kann dabei bevorzugt ein- oder beidseitig der Aufnahme verlaufen und dabei seitlich, oberhalb und/oder unterhalb der Aufnahme angeordnet sein.

Bevorzugt ist die externe Führung einteilig mit der Aufnahme bereitgestellt, wobei bevorzugt der Verbindungsbereich des Auszugskörpers in der Führung geführt ist. Auch können Magnete zur Ausbildung der Führung bereitgestellt werden. Diese sind dann beidseitig des Auszugskörpers angeordnet und wirken abstoßend auf einen magnetischen Bereich des Auszugskörpers. Die abstoßende Wirkung wird dabei durch eine jeweils gleichpolige Ausrichtung der Magnete der externen Führung bezüglich der Polung der Magnete des Auszugskörpers erreicht.

Ferner kann dadurch, dass der Scherkörper über eine externe Führung geführt ist, eine besonders stabile Führung bereitgestellt werden, da sich die Führung über einen großen Bereich der Aufnahme beziehungsweise der Vorrichtung erstrecken kann.

In einer weiter bevorzugten Ausgestaltung weist der Scherkörper zumindest ein in der Aufnahme aufgenommenes freies Ende mit einer Querschnittsverjüngung zum Reduzieren des Eintauchwiderstands des Scherkörpers in dem dilatanten Fluid auf.

Dadurch kann eine strömungsgünstige Form des freien Endes bereitgestellt werden. So ist bei einem Eindrücken des Scherkörpers in die Aufnahme ein geringerer Strömungswiderstand des Scherkörpers im dilatanten Fluid bereitgestellt.

Insbesondere, wenn die Querschnittsverjüngung des freien Endes keilförmig oder kegelförmig ausgebildet ist, kann ein besonders geringer Eindrückwiderstand bereitgestellt werden, wobei die Keilform oder Kegelform zusätzlich leicht zu fertigen ist.

In einer Weiterbildung weist die Aufnahme eine Querschnittsverengung auf. Bevorzugt ist die Querschnittsverengung im Bodenbereich der Aufnahme angeordnet. Dadurch, dass die Aufnahme eine Querschnittsverengung aufweist, kann eine Verstärkung des dämpfenden Effekts beim Eindrücken des Scherkörpers ab einem im Zuge der Auslegung der Vorrichtung beziehungsweise eines die Vorrichtung aufweisenden Medizinprodukts, wie eine Bandage oder Orthese, definierten Hubs des Scherkörpers bereitgestellt werden. So kann eine hohe Leichtgängigkeit der Vorrichtung im Bereich außerhalb der Querschnittsverengung und eine abnehmende Leichtgängigkeit des Scherkörpers beziehungsweise eine erhöhte Dämpfungswirkung im Bereich der Querschnittsverengung bereitgestellt werden, wodurch eine insbesondere zur Anwendung bei Orthopädieprodukten für sportliche Betätigung besonders geeignete Vorrichtung bereitgestellt werden kann. Bevorzugt ist die Querschnittsverengung konisch oder in Form einer abrupten Querschnittsverengung bereitgestellt.

In einer weiter bevorzugten Ausführungsvariante weist der Scherkörper mindestens ein Projektionselement zum Vergrößern der projizierten Fläche des Scherkörpers bezüglich der Eindrückrichtung des Scherkörpers in dem dilatanten Fluid auf, wobei das mindestens eine Projektionselement über ein Gelenk mit dem Scherkörper verbunden ist.

Dadurch kann eine variierende Dämpfungswirkung der Vorrichtung beim Eindrücken des Scherkörpers in die Aufnahme ermöglicht werden. Das mindestens eine Projektionselement ist dabei in Ruhelage bevorzugt in Eindrückrichtung weisend an dem Scherkörper angeordnet. Bei einem Eindrücken des Scherkörpers in die Aufnahme bewegt sich das mindestens eine Projektionselement ab einer im Zuge der Auslegung festgelegten Eindrückgeschwindigkeit aus seiner Ruhelage, so dass die projizierte Fläche bezüglich der Eindrückrichtung des Scherkörpers im Vergleich zu einem Scherkörper, bei welchem das mindestens eine Projektionselement in seiner Ruhelage vorliegt, vergrößert ist.

Ein definiertes Ausklappen beziehungsweise Schwenken des mindestens einen Projektionselements in und aus einer Ruhelage kann insbesondere dann bereitstellt werden, wenn das mindestens eine Projektionselement über ein Gelenkmit dem Scherkörper verbunden ist. Bevorzugt ist das Gelenk hierbei als Filmgelenk ausgestaltet. Alternativ können auch andere Gelenkformen Anwendung finden.

Ferner ist das mindestens eine Projektionselement derart an dem Scherkörper angeordnet, dass bei einem Ausziehen des Scherkörpers aus der Aufnahme das mindestens eine Projektionselement in seine Ruhelage zurückkehrt beziehungsweise zurückschwenkt, was durch das dilatante Fluid unterstützt wird.

In einer besonders bevorzugten Ausführungsform weist der Scherkörper mindestens ein Wirkelement zum Variieren einer scherkrafteinleitenden Fläche des Scherkörpers auf. Dadurch kann eine variierende Wirkfläche zum Einleiten beziehungsweise Übertragen der Scherkraft am Scherkörper bereitgestellt werden.

Bevorzugt sind mehrere Wirkelemente an dem Scherkörper in Form von am Scherkörper angeordneten Öffnungen oder in Eindrückrichtung am Scherkörper angeordneten Ausprägungen, bevorzugt Lamellen, angeordnet. Hierdurch können an dem Scherkörper Strömungskanäle bereitgestellt werden, durch welche das dilatante Fluid bei einem Bewegen des Scherkörpers unterhalb der kritischen Geschwindigkeit strömen kann. Ab einer bestimmten Strömungsgeschwindigkeit in diesen Strömungskanälen kommt es dort zu einer Scherverfestigung des dilatanten beziehungsweise scherverdickende Wirkmediums. Das in den Strömungskanälen scherverfestigte dilatante Fluid weist somit im Wesentlichen Feststoffcharakter auf und trägt zur Scherkrafteinleitung beziehungsweise -übertragung durch den Scherkörper bei. Hierdurch wird die Wirkfläche des Scherkörpers zum Einleiten beziehungsweise Übertragen der Scherkraft in das dilatante Fluid vergrößert beziehungsweise der dem dilatanten Fluid zur Verfügung stehende Durchflussquerschnitt zwischen der Aufnahme und dem Scherkörper verringert.

In einer besonders bevorzugten Ausführungsform ist die Aufnahme in mindestens eine Ausgleichskammer zum Ausgleichen des Volumens des dilatanten Fluids und eine Scherkammer zur Aufnahme des Scherkörpers unterteilt, wobei die mindestens eine Ausgleichskammer zumindest eine Ausgleichsöffnung aufweist, durch welche die mindestens eine Ausgleichskammer mit der Scherkammer fluidtechnisch verbunden ist.

Dadurch kann eine weiter verbesserte Leichtgängigkeit der Vorrichtung in Bereich physiologischer Bewegungen bereitgestellt werden. So muss das dilatante Fluid, welches bei einem Eindrücken des Scherkörpers in die Aufnahme von diesem verdrängt wird, nicht komplett in dem Spalt zwischen dem Scherkörper und der Innenwand der Aufnahme hindurchströmen, sondern kann zumindest teilweise durch die Ausgleichsöffnung in die mindestens eine Ausgleichskammer ausweichen. Hierdurch weist das dilatante Fluid in dem Spalt zwischen dem Scherkrafteinleitkörper und der Innenwand der Aufnahme eine reduzierte Strömungsgeschwindigkeit auf.

Bei Überschreitung einer kritischen Schergeschwindigkeit des dilatanten Fluids in der Ausgleichsöffnung wird diese aufgrund der dort auftretenden Scherverfestigung des dilatanten Fluids blockiert, so dass kein weiteres dilatantes Fluid mehr durch die Ausgleichsöffnung strömen kann. Dies bewirkt wiederum einen sprunghaften Anstieg der Strömungsgeschwindigkeit in dem Spalt, da nun das gesamte verdrängte dilatante Fluid durch den Spalt strömt. So kann ab einer im Zuge der Auslegung vorbestimmten Eindrückgeschwindigkeit des Scherkörpers eine weiter verstärkte Rückhaltekraft der Vorrichtung und gleichzeitig unterhalb der festgelegten Eindrückgeschwindigkeit eine besonders leichtgängige Vorrichtung bereitgestellt werden.

In einer bevorzugten alternativen Ausführungsform sind mindestens zwei Ausgleichsöffnungen vorgesehen, wobei mindestens eine Ausgleichsöffnung bevorzugt nahe des Bodens der Aufnahme angeordnet ist und mindestens eine Ausgleichsöffnung nahe der Einführöffnung der Aufnahme angeordnet ist. So kann durch eine der Ausgleichsöffnungen in die Ausgleichskammer strömendes dilatantes Fluid durch die andere Ausgleichsöffnung zurück in die Aufnahme strömen.

In einer bevorzugten Weiterbildung weist die Ausgleichsöffnung eine Ventileinrichtung zum Verändern des Durchflussquerschnitts der Ausgleichsöffnung auf. Dadurch kann die Geschwindigkeit, bei welcher es in der Ausgleichsöffnung zur Scherverfestigung kommt, variiert werden. Dies ermöglicht eine Vorrichtung mit variabler kritischer Eindrückgeschwindigkeit. Ferner kann die Ventileinrichtung auch zum Zu- oder Abschalten der Ausgleichskammer verwendet werden.

Insbesondere, wenn die Ventileinrichtung zum Zu- oder Abschalten der Ausgleichskammer verwendet wird, kann zwischen einer bei zugeschalteter Ausgleichskammer leichtgängigen Vorrichtung und durch Schließen der Ventileinrichtung und somit Abschalten der Ausgleichskammer einer schwergängigen Vorrichtung gewechselt werden. So kann je nach erforderlicher Schutzwirkung des Gelenks eine hohe Leichtgängigkeit mit einem entsprechend großen Bewegungsumfang bei geöffneter Ventileinrichtung sowie eine hohe Schwergängigkeit mit entsprechend hoher Schutzwirkung bei geschlossener Ventileinrichtung bereitgestellt werden. Bevorzugt erfolgt das zum Zu- oder Abschalten der Ausgleichskammer beziehungsweise das Öffnen und Schließen der Ventileinrichtung mittels eines Hebels, eines Schalters oder dergleichen.

Die oben beschriebene Vorrichtung ist insbesondere zum Einsatz an Medizinprodukten, bevorzugt Orthopädieprodukten, Sportprodukten, Protektoren, Schuhen, Schutzkleidung und/oder Fitnessausrüstung, bevorzugt Sportgeräte und/oder Polsterungen, geeignet.

Bevorzugt weisen die Bauteile der Vorrichtung, besonders bevorzugt der Scherkörper und/oder die Aufnahme, Polyamide, bevorzugt Polyethylen, Polypropylen, thermoplastische Elastomere, Polystyrol, Polymethylmethacrylat, Acrylnitril-Butadien-Styrol, Polyurethan, und/oder Duromere und/oder Elastomere auf. Alternativ können auch Silikone oder Metalle und/oder Metalllegierungen, bevorzugt Aluminium und/oder Stahl, enthalten sein.

Um erhöhte Steifigkeiten und Festigkeiten bereitzustellen, können die Bauteile der Vorrichtung bevorzugt auch verstärkende Stoffe, wie anorganische Stoffe mit Glasfasern beziehungsweise verstärkte Stoffe, bevorzugt verstärkte Kunststoffe, aufweisen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine perspektivische Ansicht einer Vorrichtung zum Begrenzen einer Bewegung eines zwischen einem ersten Körperteil und einem zweiten Körperteil angeordneten Gelenks;
- Figur 2: schematisch eine Schnittansicht durch die Mittelebene der Vorrichtung aus Figur 1;
- Figur 3: schematisch die Vorrichtung der vorstehenden Figuren in einer Schnittansicht entlang der Schnittlinie A-A aus Figur 2;
- Figur 4: schematisch eine perspektivische Ansicht einer Vorrichtung zum Begrenzen einer Bewegung eines zwischen einem ersten Körperteil und einem zweiten Körperteil angeordneten Gelenks in einer alternativen Ausführungsform;
- Figur 5: schematisch eine Schnittansicht der Vorrichtung aus Figur 4;
- Figur 6: schematisch einen Schnitt einer Detailansicht einer Aufnahme und eines Scherkörpers mit zwei Projektionselementen in Ruhelage;
- Figur 7: schematisch einen Schnitt der Detailansicht aus Figur 5 in einer von der Ruhelage abweichenden Position;
- Figur 8: schematisch einen Schnitt einer Detailansicht der Vorrichtung mit einer stufenförmigen Querschnittsverengung der Aufnahme;
- Figur 9: schematisch einen Schnitt einer Detailansicht der Vorrichtung mit einer konischen Querschnittsverengung der Aufnahme; und
- Figur 10: schematisch einen Schnitt einer Detailansicht der Vorrichtung mit einer in eine Scherkammer und eine Ausgleichskammer unterteilte Aufnahme.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf die wiederholte Beschreibung dieser Elemente in der nachfolgenden Beschreibung teilweise verzichtet.

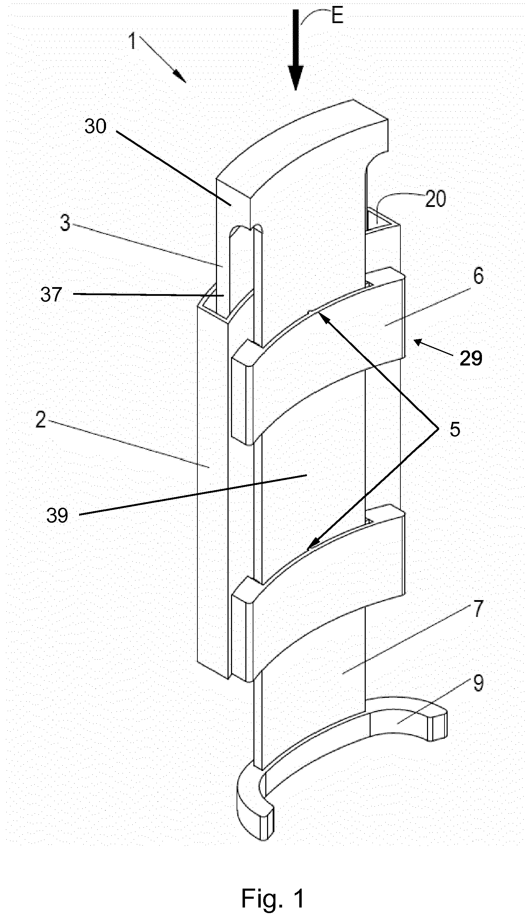

Figur 1 zeigt schematisch eine perspektivische Ansicht einer Vorrichtung 1 zum Begrenzen einer Bewegung eines zwischen einem ersten Körperteil und einem zweiten Körperteil angeordneten Gelenks. Dabei umfasst die Vorrichtung 1 eine mit einem dilatanten beziehungsweise scherverdickenden Wirkmedium in Form eines dilatanten Fluids 4 gefüllte Aufnahme 2, welche mittels eines ersten Anbindungsbereichs 6 einer Aufnahmebefestigung 29 zum Anbringen an einem ersten Körperteil angebracht werden kann, und einen Scherkörper 3, welcher mittels eines zweiten Anbindungsbereichs 7 einer Scherkörperbefestigung 9 zum Anbringen an einem zweiten Körperteil angebracht ist. Der Scherkörper 3 ist in einer Eindrückrichtung E relativ zur Aufnahme 2 beweglich und über eine Einführöffnung 20 teilweise in die Aufnahme 2 eingeführt, so dass zwischen der Aufnahme 2 und dem Scherkörper 3 ein mit dem dilatanten Fluid 4 gefüllter Raum bereitgestellt ist. Die Einführöffnung 20 ist hierbei von der Scherkörperbefestigung 9 und dem zweiten Anbindungsbereich 7 weg gerichtet.

Alternativ kann das dilatante Fluid 4 auch als Paste und/oder als ein Wirkmedium mit Feststoffcharakter, wie einem Schaum, bevorzugt einem Polymerschaum, bereitgestellt sein.

Ein U-förmiger Verbindungsbereich 30 des Scherkörpers 3 verbindet dabei einen Interaktionsabschnitt 37, der mit dem dilatanten Fluid 4 in Wirkverbindung steht, und einen Führungsabschnitt 39, welcher sich zur Scherkörperbefestigung 9 erstreckt und über welchem Kräfte und Bewegungen, die über die Scherkörperbefestigung 9 eingeleitet werden, als Zugkräfte zum Verbindungsbereich 30 übertragen werden. Über den Verbindungsbereich 30 werden diese Kräfte umgelenkt und über den Interaktionsabschnitt 37 als Druckkräfte in das dilatante Fluid 4 eingebracht.

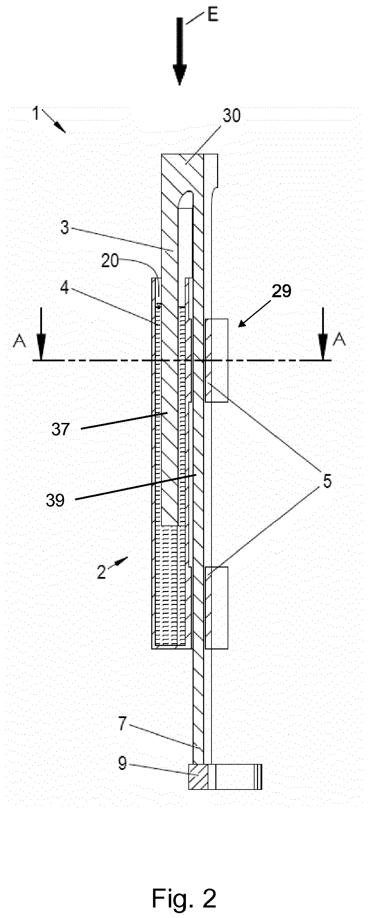

Figur 2 zeigt schematisch eine Schnittansicht durch die Mittelebene parallel zur Eindrückrichtung E der Vorrichtung 1 aus Figur 1. Der Scherkörper 3 ist in Eindrückrichtung E relativ zur Aufnahme 2 beweglich und über eine Einführöffnung 20 teilweise in die Aufnahme 2 eingeführt, so dass zwischen der Aufnahme 2 und dem Scherkörper 3 ein mit dem dilatanten Fluid 4 gefüllter Raum bereitgestellt ist.

Die Einführöffnung 20 ist dabei auf der vom zweiten Anbindungsbereich 7 abgewandten Seite der Aufnahme 2 angeordnet. Zur Verbindung des aus der Einführöffnung 20 heraustretenden Scherkörpers 3 mit dem zweiten Anbindungsbereich 7 weist der Scherkörper 3 den U-förmigen Verbindungsbereich 30 auf, welcher den Scherkörper 3 in den Interaktionsabschnitt 37 und den Führungsabschnitt 39 welcher außerhalb des mit dem dilatanten Fluid 4 gefüllten Raums der Aufnahme 2 an dieser vorbei über den zweiten Anbindungsbereich 7 zur Scherkörperbefestigung 9 verläuft, teilt. Hierdurch wird der Scherkörper 3 bei einem Auseinanderbewegen der Aufnahmebefestigung 29 und der Scherkörperbefestigung 9 in die Aufnahme 2 bewegt.

Zum Abdichten des mit dem dilatanten Fluid 4 gefüllten Raums der Aufnahme 2 ist an der Einführöffnung 20 zwischen der Aufnahme 2 und dem Scherkörper 3 eine hier nicht gezeigte Dichtung angeordnet. Im vorliegenden Fall ist diese aus einem elastischen Material bereitgestellt und fest mit der Aufnahme 2 und dem Scherkörper 3 verbunden.

In diesem Ausführungsbeispiel ist der Scherkörper 3 aus Polyamid und die Aufnahme 2 aus Polyurethan hergestellt. Alternativ können die beiden Bauteile auch aus anderen Kunststoffen, wie Silikon, Gummi oder bevorzugt einem anderen thermoplastischen Material wie beispielsweise Polyamid, Polypropylen, Polyethylen oder Polyurethan bestehen, wobei insbesondere bei der Aufnahme 2 darauf zu achten ist, dass das verwendete Material fluidundurchlässig ist.

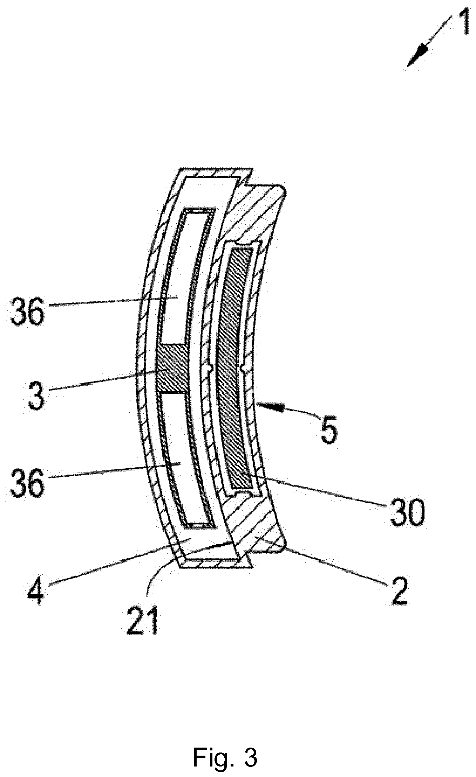

In Figur 3 ist schematisch die Vorrichtung 1 der vorstehenden Figuren in einer Schnittansicht entlang der Schnittlinie A-A aus Figur 2 gezeigt. Der Scherkörper 3 und die Innenwand 21 der Aufnahme 2 weisen einen konstanten Abstand auf. Um diesen Abstand einzuhalten, ist der Scherkörper 3 an dessen Führungsabschnitt 39 in der externen Führung 5 der Aufnahme 2 geführt.

An dem Scherkörper 3 sind Wirkelemente 36 in Form von Strömungskanälen bereitgestellt, durch welche das dilatante Fluid 4 bei einem Bewegen des Scherkörpers 3 unterhalb einer kritischen Schergeschwindigkeit strömen kann. Ab der kritischen Strömungsgeschwindigkeit kommt es in diesen Strömungskanälen zu einer Scherverfestigung des dilatanten Fluids 4, was den Eindrückwiderstand des Scherkörpers in dem dilatanten Fluid 4 sprunghaft erhöht.

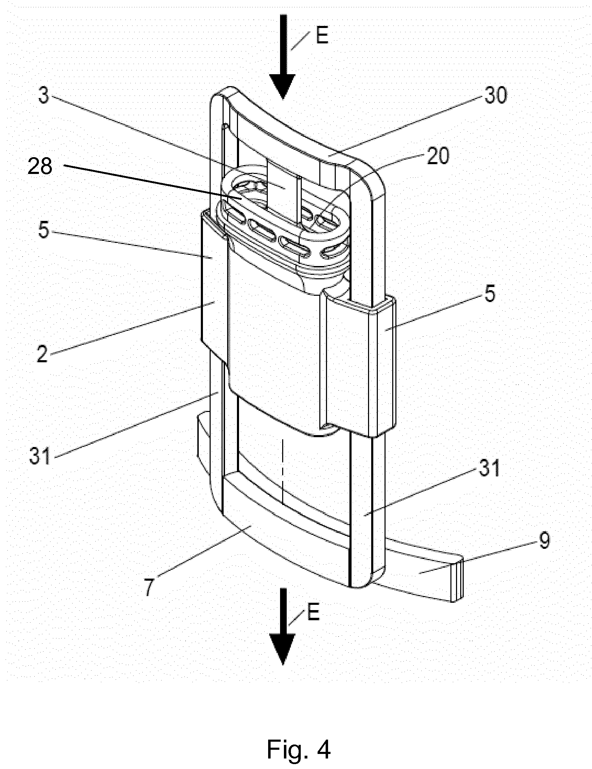

Figur 4 zeigt schematisch eine perspektivische Ansicht einer Vorrichtung 1 zum Begrenzen einer Bewegung eines zwischen einem ersten Körperteil und einem zweiten Körperteil angeordneten Gelenks in einer alternativen Ausführungsform, wobei die Vorrichtung 1 analog zur Vorrichtung aus den Figuren 1 bis 3 eine mit dilatantem Fluid 4 gefüllte Aufnahme 2 und einen relativ zur Eindrückrichtung E bewegbaren Scherkörper 3 aufweist, welcher über eine Einführöffnung 20 teilweise in die Aufnahme 2 eingeführt ist. Um eine besonders flache Vorrichtung 1 bereitzustellen, ist der u-förmige Verbindungsbereich 30 in zwei Verbindungsarme 31 unterteilt, welche seitlich an der Aufnahme 2 jeweils in einer externen Führung 5 geführt sind. An den Enden der Verbindungsarme 31 gehen diese in den zweiten Anbindungsbereich 7 über, welcher an die Scherkörperbefestigung 9 angebunden ist. Der erste Anbindungsbereich ist direkt an der Rückseite der Aufnahme 2 angeordnet.

Sowohl die Aufnahme 2, als auch der Scherkörper 3 sind aus einem thermoplastischen Polyurethan hergestellt. Zur Anbindung einer Dichtung im Bereich der Einführöffnung 20 weist die Aufnahme einen Kranz 28 auf. An diesem wird im vorliegenden Beispiel eine Dichtung aus einem thermoplastischen Elastomer gegossen. Alternativ können zum Abdichten des mit dem dilatanten Fluid 4 gefüllten Raums der Aufnahme 2 auch andere Materialien, bevorzugt hygienetaugliche Materialien, Anwendung finden.

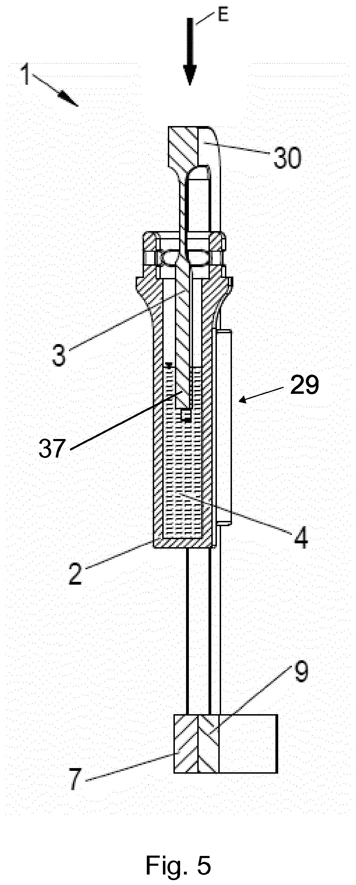

Figur 5 zeigt schematisch eine Schnittansicht der Vorrichtung aus Figur 4 in einer Mittelschnittansicht. Die Aufnahme 2 ist in diesem Ausführungsbeispiel lediglich teilweise mit dilatantem Fluid 4 befüllt, so dass der Interaktionsabschnitt 37 des Scherkörpers 3 nur teilweise mit dem dilatanten Fluid 4 in Kontakt kommt. Mit zunehmender Eindrücktiefe des Scherkörpers 3 in die Aufnahme 2 und damit einhergehender zunehmender Eintauchtiefe in das dilatante Fluid 4 vergrößert sich die vom Scherkörper 3 effektiv zur Scherkraftübertragung genutzte Fläche. Dadurch weist die Vorrichtung 1 eine extreme Leichtgängigkeit auf, wenn der Scherkörper 3 nicht oder nur in geringem Maße in das dilatante Fluid 4 eintaucht. Ferner nehmen die dämpfende Wirkung und ebenso die Rückhaltekraft der Vorrichtung 1 gegen ein Auseinanderbewegen der Aufnahmebefestigung 29 und der Scherkörperbefestigung 9 mit zunehmender Eintauchtiefe des Scherkörpers 3 zu.

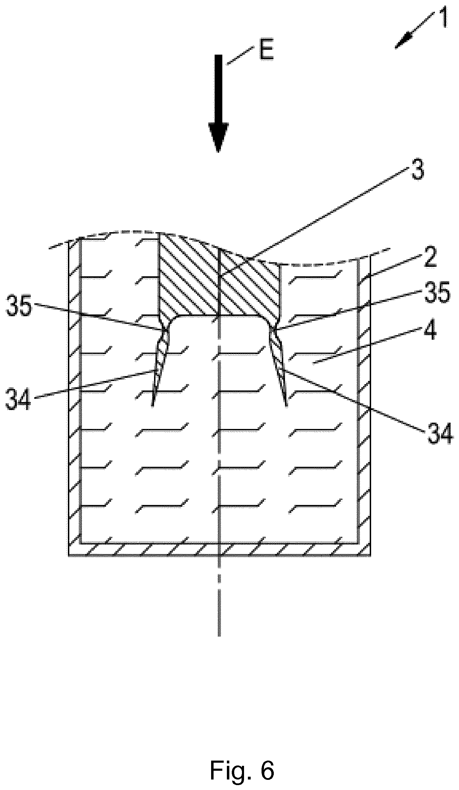

Figur 6 zeigt schematisch eine Schnittansicht einer Vorrichtung 1 zum Begrenzen einer Bewegung eines zwischen einem ersten Körperteil und einem zweiten Körperteil angeordneten Gelenks mit zwei Projektionselementen 34 in Ruhelage. Die Projektionselemente 34 sind an dem in die Aufnahme 2 eingeführten freien Ende des Scherkörpers 3 angeordnet und weisen in Richtung der Eindrückrichtung E. An den Verbindungsstellen zwischen den Projektionselementen 34 und dem Scherkörper 3 weisen die Projektionselemente 34 jeweils ein Filmgelenk 35 auf. Die Filmgelenke 35 sind dabei so dimensioniert, dass die zwei Projektionselemente 34 bei moderaten Bewegungen des Scherkörpers 3 und damit einhergehenden langsamen Eindrückbewegungen des Scherkörpers 3 im Wesentlichen in der Ruhelage bleiben.

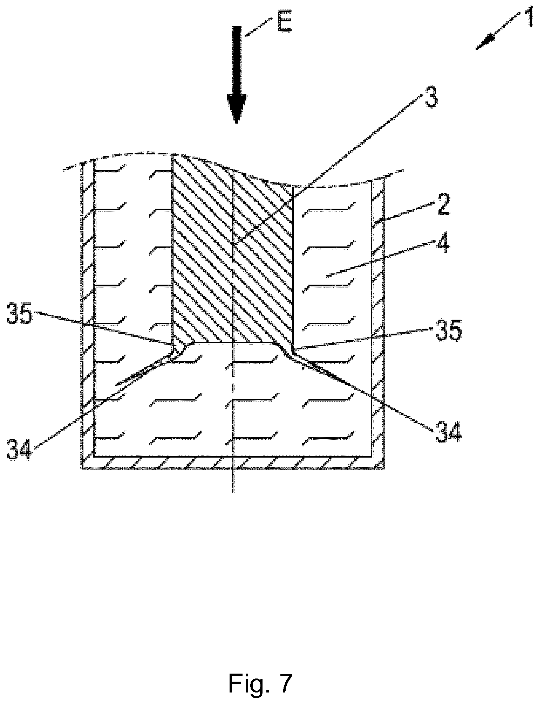

Figur 7 zeigt schematisch eine Schnittansicht der Vorrichtung 1 aus Figur 5 in einer von der Ruhelage abweichenden Position. Die zwei Projektionselemente 34 sind aufgrund deren asymmetrischer Form und der daraus resultierenden asymmetrischen Anströmung beim Eindrücken mit hoher Geschwindigkeit, welche bei unphysiologischen Bewegungen auftritt, aus der Ruhelage um die Filmgelenke 35 seitlich ausgeschwenkt. Dadurch ist die projizierte Fläche des Scherkörpers 3 senkrecht zur Eindrückrichtung E im Vergleich zur projizierten Fläche des Scherkörpers 3 mit Projektionselementen 34 in Ruhelage vergrößert, was einen vergrößerten Eindrückwiderstand des Scherkörpers 3 in dem dilatanten Fluid 4 bewirkt. Dies hat einen verstärkten dämpfenden Effekt der Vorrichtung beim Eindrücken zur Folge, welcher zum einen auf der vergrößerten projizierten Fläche und dem damit einhergehenden vergrößerten Strömungswiderstand und zum anderen auf der hierdurch verstärken Scherbelastung auf das dilatante Fluid 4 beruht.

Bei einer Auszugbewegung des Scherkörpers 3 beziehungsweise sobald die Eindrückgeschwindigkeit einen im Zuge der Auslegung festgelegten Wert unterschreitet, schwenken die Projektionselemente 34 zurück in Richtung ihrer Ruhelage.

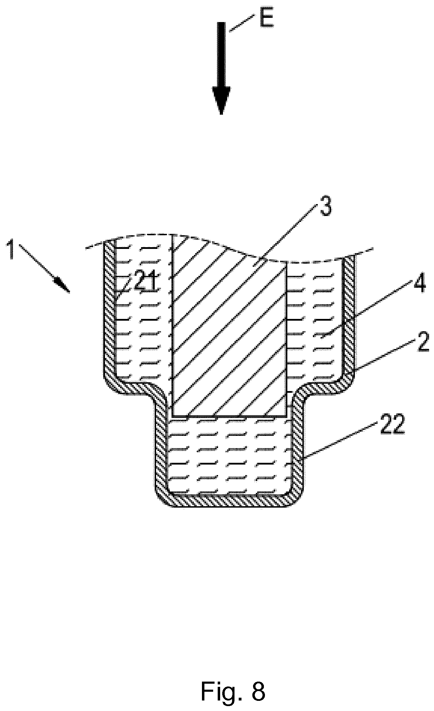

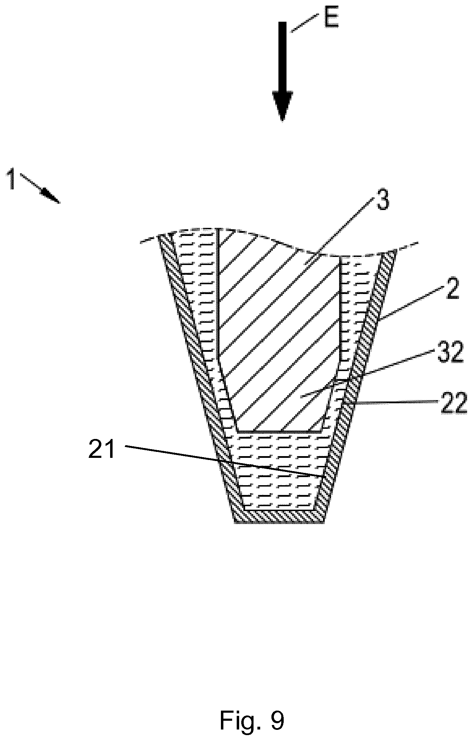

Aus Figur 8 ist schematisch eine Schnittansicht einer Vorrichtung 1 zum Begrenzen einer Bewegung eines zwischen einem ersten Körperteil und einem zweiten Körperteil angeordneten Gelenks mit einer Aufnahme 2, welche eine Querschnittsverengung 22 aufweist, zu entnehmen. Im Bereich außerhalb der Querschnittsverengung 22 ist zwischen dem Scherkörper 3 und der Innenwand 21 der Aufnahme 2 aufgrund deren breiten Querschnitts ein vergleichsweise großer Abstand bereitgestellt. Im Bereich der Querschnittsverengung 22 ist dieser Abstand aufgrund des verengten Querschnitts der Aufnahme 2 geringer, so dass dort bei gleicher Eindrückgeschwindigkeit des Scherkörpers 3 eine vergrößerte Scherung auf das dilatante Fluid 4 wirkt. Die Vorrichtung 1 ist dabei so ausgelegt, dass sich der Scherkörper 3 in einem physiologischen Winkelbereich des zu schützenden Gelenks im Bereich außerhalb der Querschnittsverengung 22 bewegt und somit eine vergleichsweise hohe Leichtgängigkeit aufweist. Ab Überschreiten eines kritischen Gelenkwinkels beziehungsweise eines kritischen Hubs des Scherkörpers 3, welcher im Zuge der Auslegung festgelegt werden kann, wird der Scherkörper 3 in den Bereich der Querschnittsverengung 22 eingedrückt. Durch den geringeren Spalt kommt es dann im Bereich der Querschnittsverengung 22 zu einer hohen Scherrate des dilatanten Fluids 4, wodurch der Scherkörper 3 in seiner Bewegung gedämpft, gebremst und/oder abgestoppt wird. Figur 9 zeigt schematisch eine Schnittansicht einer weiteren Vorrichtung 1 zum Begrenzen einer Bewegung eines zwischen einem ersten Körperteil und einem zweiten Körperteil angeordneten Gelenks mit einer Querschnittsverengung 22 der Aufnahme 2 in einer weiteren Ausführungsform, wobei sich der Querschnitt der Aufnahme 2 kontinuierlich verjüngt. Die Querschnittsverengung 22 ist hierbei konisch bereitgestellt. Ferner weist der Scherkörper 3 auf seiner in das dilatante Fluid 4 eintauchenden Seite eine keilförmige Querschnittsverjüngung 32 auf. Im vorliegenden Fall sind die Flanken der Querschnittsverjüngung 32 im Wesentlichen parallel zu den konischen Innenwänden 21 der Aufnahme 2. So kann im Bereich der Querschnittsverjüngung 32 zwischen dem Scherkörper 3 und der Innenwand 21 der Aufnahme 2 ein gleichmäßiger Spalt bereitgestellt werden, welcher sich mit zunehmender Eindrücktiefe des Scherkörpers 3 verringert, wodurch ein bezüglich der Eindrücktiefe progressiver Scherbelastungsverlauf auf das dilatante Fluid 4 bereitgestellt werden kann.

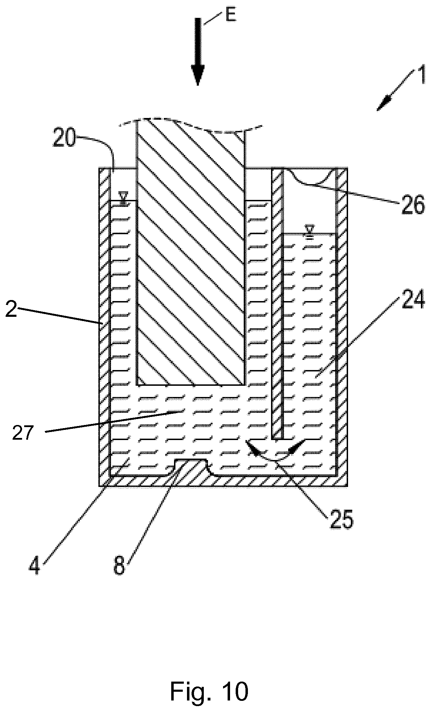

In Figur 10 ist schematisch eine Schnittansicht einer Vorrichtung 1 zum Begrenzen einer Bewegung eines zwischen einem ersten Körperteil und einem zweiten Körperteil angeordneten Gelenks mit einer an der Aufnahme 2 angeordneten Ausgleichskammer 24 gezeigt. Zwischen der Ausgleichskammer 24 und der Aufnahme 2 ist eine Ausgleichsöffnung 25 angeordnet. Durch diese Ausgleichsöffnung 25 kann dilatantes Fluid 4 aus der Aufnahme 2 in die Ausgleichskammer 24 und umgekehrt strömen. Wird der Scherkörper 3 in die Aufnahme 2 gedrückt, weicht das durch den Scherkörper 3 verdrängte Volumen des dilatanten Fluids 4 zumindest teilweise in die Ausgleichskammer 24 aus. Zum Abdichten der Ausgleichskammer 24 an der der Aufnahmebefestigung zugewandten Seite der Aufnahme 2 ist dort eine elastische Ausgleichsmembran 26 angeordnet. Ferner fungiert die Ausgleichsmembran 26 zum Ausgleichen der in der Ausgleichskammer 24 infolge der variierenden Pegelstände des dilatanten Fluids 4 entstehenden Druckunterschiede zwischen der Ausgleichskammer und der Umgebung. Alternativ kann die Ausgleichskammer 24 auch geschlossen bereitgestellt werden, wobei ein in der Ausgleichskammer 24 eingeschlossenes Gas- und/oder Gasgemischvolumen als Dämpfungselement dienen kann.

Alternativ kann auch im Bereich der Einführöffnung 20 eine zweite Ausgleichsöffnung vorgesehen sein, durch welche das in die Ausgleichskammer 24 durch die erste Ausgleichsöffnung 25 strömende dilatante Fluid 4 wieder in die Aufnahme 2 strömen kann und umgekehrt.

Da das dilatante Fluid 4, welches bei einem Eindrücken des Scherkörpers 3 in die Aufnahme 2 von diesem verdrängt wird, nicht komplett in dem Spalt zwischen dem Scherkrafteinleitkörper 3 und der Innenwand 21 der Aufnahme 2 hindurchströmt, sondern zumindest teilweise durch die Ausgleichsöffnung in die mindestens eine Ausgleichskammer ausweicht, ist die Vorrichtung 1 im Bereich physiologischer Bewegungen besonders leichtgängig.

Bei Überschreiten einer kritischen Schergeschwindigkeit des dilatanten Fluids 4 in der Ausgleichsöffnung 25 wird diese aufgrund der dort auftretenden Scherverfestigung des dilatanten Fluids 4 blockiert, so dass kein weiteres dilatantes Fluid 4 mehr durch die Ausgleichsöffnung 25 in die Ausgleichskammer 24 strömen kann und sich die Strömungsgeschwindigkeit des dilatanten Fluids 4 somit in dem Spalt zwischen dem Scherkrafteinleitkörper 3 und der Innenwand 21 der Aufnahme 2 erhöht, da nun das gesamte verdrängte dilatante Fluid 4 durch den Spalt strömt. Hierdurch entsteht ab einer im Zuge der Auslegung vorbestimmten Eindrückgeschwindigkeit des Scherkörpers 3 eine weiter verstärkte Rückhaltekraft der Vorrichtung 1. So weist die Vorrichtung 1 in dieser Ausführungsform trotz der hohen Leichtgängigkeit im physiologischen Bereich eine starke bewegungsbegrenzende Wirkung aufgrund der starken Scherung des dilatanten Fluids 4 ab der vorbestimmten Eindrückgeschwindigkeit auf.

Am Boden der Aufnahme 2 ist ein Anschlag 8 angeordnet, welcher zum Begrenzen der Einführbewegung des Scherkörpers 3 in die Aufnahme 2 vorgesehen ist.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung
- 2: Aufnahme
- 20: Einführöffnung
- 21: Innenwand
- 22: Querschnittsverengung
- 24: Ausgleichskammer
- 25: Ausgleichsöffnung
- 26: Ausgleichsmembran
- 27: Scherkammer
- 28: Kranz
- 29: Aufnahmebefestigung
- 3: Scherkörper
- 30: Verbindungsbereich
- 31: Verbindungsarm
- 32: Querschnittsverjüngung
- 34: Projektionselement
- 35: Filmgelenk
- 36: Wirkelement
- 37: Interaktionsabschnitt
- 39: Führungsabschnitt
- 4: Dilatantes Fluid
- 5: Externe Führung
- 6: Erster Anbindungsbereich
- 7: Zweiter Anbindungsbereich
- 8: Anschlag
- 9: Scherkörperbefestigung

- E: Eindrückrichtung
- A-A: Schnittlinie

## Patentansprüche

1. Vorrichtung (1) zum Begrenzen einer Bewegung eines zwischen einem ersten Körperteil und einem zweiten Körperteil angeordneten Gelenks, umfassend eine mittels einer Aufnahmebefestigung (29) an dem ersten Körperteil gehaltene Aufnahme (2), einen mittels einer Scherkörperbefestigung (9) an dem zweiten Körperteil gehaltenen, relativ zu der Aufnahme (2) bewegbaren Scherkörper (3), wobei der Scherkörper (3) über eine Einführöffnung (20) zumindest teilweise in die Aufnahme (2) eingeführt ist, und ein in der Aufnahme (2) bereitgestelltes dilatantes Fluid (4) zur Beeinflussung einer Relativbewegung zwischen dem Scherkörper (3) und der Aufnahme (2),
**dadurch gekennzeichnet, dass**
die Einführöffnung (20) der Aufnahme (2) von der Scherkörperbefestigung (9) weg gerichtet ist.

2. Vorrichtung (1) gemäß Anspruch 1, **dadurch gekennzeichnet, dass** zwischen dem Scherkörper (3) und der Scherkörperbefestigung (9) ein U-förmiger Verbindungsbereich (30) zum Verbinden des Scherkörpers (3) mit der Scherkörperbefestigung (9) angeordnet ist.

3. Vorrichtung (1) gemäß Anspruch 1 oder 2, **dadurch gekennzeichnet, dass** die Aufnahmebefestigung (29) und die Scherkörperbefestigung (9) derart angeordnet sind, dass bei an das erste Körperteil angebrachter Aufnahmebefestigung (29) und bei an das zweite Körperteil angebrachter Scherkörperbefestigung (9) die Einführöffnung (20) proximal oder kranial an der Aufnahme (2) angeordnet ist.

4. Vorrichtung gemäß einem der vorstehenden Ansprüche, **dadurch gekennzeichnet, dass** der U-förmige Verbindungsbereich (30) einen Interaktionsabschnitt (37) des Scherkörpers (3), der mit dem dilatanten Fluid (4) in Wirkverbindung steht, und einen Führungsabschnitt (39) des Scherkörpers (3), welcher sich zur Scherkörperbefestigung (9) erstreckt, verbindet, wobei der Interaktionsabschnitt (37) und der Führungsabschnitt (39) sich zumindest teilweise parallel und voneinander beabstandet in Eindrückrichtung (E) erstrecken und sich zumindest teilweise überlappen.

5. Vorrichtung (1) gemäß einem der vorstehenden Ansprüche, **dadurch gekennzeichnet, dass** ein Anschlag (8) zum Begrenzen der Einführbewegung des Scherkörpers (3) in die Aufnahme (2) vorgesehen ist.

6. Vorrichtung gemäß einem der Ansprüche 1 bis 4, **dadurch gekennzeichnet, dass** der Anschlag (8) am U-förmigen Verbindungsbereich (30) angeordnet und/oder durch diesen ausgebildet ist.

7. Vorrichtung (1) gemäß einem der vorstehenden Ansprüche, **dadurch gekennzeichnet, dass** der Scherkörper (3) über eine externe Führung (5) geführt ist, wobei die externe Führung (5) außerhalb eines mit dem dilatanten Fluid (4) gefüllten Bereichs der Aufnahme (3) angeordnet ist.

8. Vorrichtung (1) gemäß einem der vorstehenden Ansprüche, **dadurch gekennzeichnet, dass** der Scherkörper (3) zumindest ein in der Aufnahme (2) aufgenommenes freies Ende mit einer Querschnittsverjüngung (32) zum Reduzieren des Eintauchwiderstands des Scherkörpers (3) in dem dilatanten Fluid (4) aufweist.

9. Vorrichtung (1) gemäß einem der vorstehenden Ansprüche, **dadurch gekennzeichnet, dass** die Aufnahme (2) eine Querschnittsverengung (22) aufweist.

10. Vorrichtung (1) gemäß einem der vorstehenden Ansprüche, **dadurch gekennzeichnet, dass** der Scherkörper (3) mindestens ein Projektionselement (34) zum Vergrößern der projizierten Fläche des Scherkörpers (3) bezüglich der Eindrückrichtung (E) des Scherkörpers (3) in dem dilatanten Fluid (4) aufweist, wobei das mindestens eine Projektionselement (34) über ein Gelenk mit dem Scherkörper (3) verbunden ist.

11. Vorrichtung (1) gemäß einem der vorstehenden Ansprüche, **dadurch gekennzeichnet, dass** der Scherkörper (3) mindestens ein Wirkelement (36) zum Variieren einer scherkrafteinleitenden Fläche des Scherkörpers (3) aufweist.

12. Vorrichtung (1) gemäß einem der vorstehenden Ansprüche, **dadurch gekennzeichnet, dass** die Aufnahme (2) in mindestens eine Ausgleichskammer (24) zum Ausgleichen des Volumens des dilatanten Fluids (4) und eine Scherkammer (27) zur Aufnahme des Scherkörpers (3) unterteilt ist, wobei die mindestens eine Ausgleichskammer (24) zumindest eine Ausgleichsöffnung (25) aufweist, durch welche die mindestens eine Ausgleichskammer (24) mit der Scherkammer (27) fluidtechnisch verbunden ist.

13. Vorrichtung (1) gemäß Anspruch 12, **dadurch gekennzeichnet, dass** die Ausgleichsöffnung (24) eine Ventileinrichtung zum Verändern des Durchflussquerschnitts der Ausgleichsöffnung (24) aufweist.

## Claims

1. Device (1) for limiting a movement of a joint arranged between a first body part and a second body part, comprising a receptacle (2) that is held on the first body part by means of a receptacle fastener (29), a shear body (3) that is held on the second body part by means of a shear-body fastener (9) and is movable relative to the receptacle (2), wherein the shear body (3) is inserted at least partially into the receptacle (2) via an insertion opening (20), and a dilatant fluid (4), provided in the receptacle (2), for influencing a relative movement between the shear body (3) and the receptacle (2),
**characterised in that**
the insertion opening (20) of the receptacle (2) is directed away from the shear-body fastener (9).

2. Device (1) according to claim 1, **characterised in that** a U-shaped connection region (30) for connecting the shear body (3) to the shear-body fastener (9) is arranged between the shear body (3) and the shear-body fastener (9).

3. Device (1) according to claim 1 or 2, **characterised in that** the receptacle fastener (29) and the shear-body fastener (9) are arranged, such that in respect of the receptacle fastener (29) attached to the first body part and in respect of the shear-body fastener (9) attached to the second body part the insertion opening (20) is arranged proximally or cranially on the receptacle (2).

4. Device according to any one of the preceding claims, **characterised in that** the U-shaped connection region (30) connects an interaction section (37) of the shear body (3), which is operatively connected to the dilatant fluid (4), and a guide section (39) of the shear body (3), which extends to the shear-body fastener (9), wherein the interaction section (37) and the guide section (39) extend at least in part parallel and spaced apart from each other in push-in direction (E) and overlap each other at least partially.

5. Device (1) according to any one of the preceding claims, **characterised in that** a stop (8) is provided for limiting the insertion movement of the shear body (3) into the receptacle (2).

6. Device according to any one of claims 1 to 4, **characterised in that** the stop (8) is arranged at the U-shaped connection region (30) and/or formed by means of this.

7. Device (1) according to any one of the preceding claims, **characterised in that** the shear body (3) is guided by way of an external guide (5), wherein the external guide (5) is arranged outside a region of the receptacle (3) filled with the dilatant fluid (4).

8. Device (1) according to any one of the preceding claims, **characterised in that** the shear body (3) has at least one free end accommodated in the receptacle (2), with a tapering of the cross-section (32) to reduce the immersion resistance of the shear body (3) in the dilatant fluid (4).

9. Device (1) according to any one of the preceding claims, **characterised in that** the receptacle (2) has a cross-section constriction (22).

10. Device (1) according to any one of the preceding claims, **characterised in that** the shear body (3) has at least one projection element (34) to enlarge the projected area of the shear body (3) with regard to the push-in direction (E) of the shear body (3) in the dilatant fluid (4), wherein the at least one projection element (34) is connected to the shear body (3) by way of a joint.

11. Device (1) according to any one of the preceding claims, **characterised in that** the shear body (3) has at least one active element (36) for varying a shear-force-inducing area of the shear body (3).

12. Device (1) according to any one of the preceding claims, **characterised in that** the receptacle (2) is divided into at least one equalisation chamber (24) for equalising the volume of the dilatant fluid (4) and a shear chamber (27) for receiving the shear body (3), wherein the at least one equalisation chamber (24) has at least one equalisation opening (25), by means of which the at least one equalisation chamber (24) is fluidically connected to the shear chamber (27).

13. Device (1) according to claim 12, **characterised in that** the equalisation opening (24) has a valve device for changing the cross-section of the flow of the equalisation opening (24).

## Revendications

1. Dispositif (1) permettant de limiter un mouvement d'une articulation agencée entre une première partie corporelle et une deuxième partie corporelle, comprenant un logement (2) retenu au niveau de la première partie corporelle au moyen d'une fixation de logement (29), un corps de cisaillement (3) mobile par rapport au logement (2), retenu au niveau de la deuxième partie corporelle au moyen d'une fixation de corps de cisaillement (9), dans lequel le corps de cisaillement (3) est introduit au moins en partie dans le logement (2) par le biais d'une ouverture d'introduction (20), et un fluide dilatant (4) fourni dans le logement (2) pour influencer un mouvement relatif entre le corps de cisaillement (3) et le logement (2),
**caractérisé en ce que**
l'ouverture d'introduction (20) du logement (2) est dirigée à l'opposé de la fixation de corps de cisaillement (9).

2. Dispositif (1) selon la revendication 1, **caractérisé en ce qu'**une zone de liaison en forme de U (30) pour relier le corps de cisaillement (3) à la fixation de corps de cisaillement (9) est agencée entre le corps de cisaillement (3) et la fixation de corps de cisaillement (9).

3. Dispositif (1) selon la revendication 1 ou 2, **caractérisé en ce que** la fixation de logement (29) et la fixation de corps de cisaillement (9) sont agencées de sorte que l'ouverture d'introduction (20) soit agencée de manière proximale ou crâniale au niveau du logement (2) lorsque la fixation de logement (29) est montée au niveau de la première partie corporelle et lorsque la fixation corps de cisaillement (9) est montée au niveau de la deuxième partie corporelle.

4. Dispositif selon l'une quelconque des revendications précédentes, **caractérisé en ce que** la zone de liaison en forme de U (30) relie une section d'interaction (37) du corps de cisaillement (3), qui est en liaison active avec le fluide dilatant (4), et une section de guidage (39) du corps de cisaillement (3), lequel s'étend vers la fixation de corps de cisaillement (9), dans lequel la section d'interaction (37) et la section de guidage (39) s'étendent au moins en partie parallèlement et espacés l'un de l'autre dans la direction d'enfoncement (E) et se chevauchent au moins en partie.

5. Dispositif (1) selon l'une quelconque des revendications précédentes, **caractérisé en ce qu'**une butée (8) est prévue pour limiter le mouvement d'introduction du corps de cisaillement (3) dans le logement (2).

6. Dispositif selon l'une quelconque des revendications 1 à 4, **caractérisé en ce que** la butée (8) est agencée au niveau de la zone de liaison en forme de U (30) et/ou est réalisée par celle-ci.

7. Dispositif (1) selon l'une quelconque des revendications précédentes, **caractérisé en ce que** le corps de cisaillement (3) est guidé par le biais d'un guidage externe (5), dans lequel le guidage externe (5) est agencé à l'extérieur d'une zone du logement (3) remplie du fluide dilatant (4).

8. Dispositif (1) selon l'une quelconque des revendications précédentes, **caractérisé en ce que** le corps de cisaillement (3) présente au moins une extrémité libre reçue dans le logement (2) avec un effilement de section transversale (32) pour la réduction de la résistance à l'immersion du corps de cisaillement (3) dans le fluide dilatant (4).

9. Dispositif (1) selon l'une quelconque des revendications précédentes, **caractérisé en ce que** le logement (2) présente un rétrécissement de section transversale (22).

10. Dispositif (1) selon l'une quelconque des revendications précédentes, **caractérisé en ce que** le corps de cisaillement (3) présente au moins un élément de projection (34) pour l'agrandissement de la surface projetée du corps de cisaillement (3) par rapport à la direction d'enfoncement (E) du corps de cisaillement (3) dans le fluide dilatant (4), dans lequel l'au moins un élément de projection (34) est relié au corps de cisaillement (3) par le biais d'une articulation.

11. Dispositif (1) selon l'une quelconque des revendications précédentes, **caractérisé en ce que** le corps de cisaillement (3) présente au moins un élément actif (36) pour faire varier une surface introduisant une force de cisaillement du corps de cisaillement (3).

12. Dispositif (1) selon l'une quelconque des revendications précédentes, **caractérisé en ce que** le logement (2) est subdivisé en au moins une chambre de compensation (24) pour la compensation du volume du fluide dilatant (4) et une chambre de cisaillement (27) pour le logement du corps de cisaillement (3), dans lequel l'au moins une chambre de compensation (24) présente au moins une ouverture de compensation (25), par laquelle l'au moins une chambre de compensation (24) est reliée de manière fluidique à la chambre de cisaillement (27).

13. Dispositif (1) selon la revendication 12, **caractérisé en ce que** l'ouverture de compensation (24) présente un dispositif de soupape pour modifier la section transversale d'écoulement de l'ouverture de compensation (24).
